(19) Europäisches Patentamt
European Patent Office
Office européen des brevets

(11) Publication number: **0 439 373 A1**

(12) **EUROPEAN PATENT APPLICATION**

(21) Application number: **91300605.2**

(22) Date of filing: **25.01.91**

(51) Int. Cl.[5]: **C09C 1/02,** // A61K47/02, A61K9/16, C08K9/08, C08K3/26, D21H19/44

(30) Priority: **26.01.90 US 471311**

(43) Date of publication of application: **31.07.91 Bulletin 91/31**

(84) Designated Contracting States: **AT BE CH DE DK ES FR GB GR IT LI LU NL SE**

(71) Applicant: **PFIZER INC.**
**235 East 42nd Street**
**New York, N.Y. 10017 (US)**
Applicant: **Lehman, Richard L.**
**107 Ridgeview Drive**
**Belle Mead, New Jersey 08502 (US)**

(72) Inventor: **Wise, Kenneth J.**
**74 Glenview Street**
**Schnecksville, Pennsylvania 18078 (US)**
Inventor: **Lehman, Richard L.**
**107 Ridgeview Drive,**
**Belle Mead, New Jersey 08502 (US)**

(74) Representative: **Wood, David John et al**
**PFIZER LIMITED, Ramsgate Road**
**Sandwich, Kent CT13 9NJ (GB)**

(54) **Low density agglomerate utilizing a carbohydrate binder.**

(57) A process for the preparation of a low density, substantially water-insoluble, particulate material which comprises forming an aqueous slurry containing precipitated calcium carbonate and a carbohydrate binder and treating said slurry to heating and mechanical processing steps to provide a particulate material having a particle size of 5-300 microns, a particle density of no more than 1.0 g/ml., and a resistance against degradation in water; and a low density, substantially water-insoluble, calcium carbonate-containing, particulate material prepared by such process.

EP 0 439 373 A1

# LOW DENSITY AGGLOMERATE UTILIZING A CARBOHYDRATE BINDER

This invention relates to a low density, substantially water-insoluble, particulate material and to a process for the preparation thereof. More particularly, the invention is concerned with a process for the preparation of a low density calcium carbonate-containing agglomerate, utilizing a carbohydrate as a binder, and the product of such process.

Calcium carbonate, commonly known as chalk, exists in nature as the minerals aragonite, calcite and vaterite and, in a precipitated or purified form, is widely used as a filler in the manufacture of paint, rubber, plastics, paper, dentifrices and other products ; and also in pharmaceuticals and cosmetics. It is also used therapeutically as an antacid, a dietary supplement and an antidiarrheal agent.

It is generally available commercially in two crystalline forms, viz : orthorhombic aragonite having a melting point of 825C. (dec.) and a density of 2.83 g/ml., and hexagonal or rhombohedral calcite having a melting point of 1339C. (102.5 atm.) and a density of 2.71 g/ml.

Presently co-pending U.S. Patent Application Serial No. 298,085 for "Low Density Agglomerate", filed January 17, 1989, also assigned to Pfizer, Inc. and incorporated herein by reference, teaches and claims a low density calcium carbonate-based agglomerate in which an alkali metal polyphosphate is utilizable as a binder, and a process for preparing such material.

Surprisingly, it has now been found that a lightweight, i.e. low density, agglomerate based on calcium carbonate and having a density of no more than 1.0 g/ml., can be prepared from precipitated calcium carbonate by subjecting the precipitated calcium carbonate, combined with a carbohydrate binder, to certain heating and mechanical processing steps.

In accordance with the present invention there is provided a process for the preparation of a low density, substantially water-insoluble, particulate material which comprises forming an aqueous slurry containing precipitated calcium carbonate having an initial average particle size of about 0.5 to 4.0 microns and 0 to 40% by weight, based on the weight of the calcium carbonate, of a dissolved carbohydrate binder, drying said slurry to provide a granular material, heating the dried material to a temperature within the range of 200 to 800°C and maintaining the temperature for a period of from about one-half to about three hours, cooling the resulting agglomerate and crushing the material to a particle size of from about 5 to about 300 microns, to provide the desired particulate material having a particle density of no more than 1.0 g/ml. and a resistance against degradation in water.

The use of a food-grade carbohydrate binder affords great flexibility for utilizing the agglomerates of this invention in situations where a high purity, non-toxic, ingestible, food-grade agglomerate is required.

Suitable food-grade carbohydrate binders which may be used according to the present invention include the class of saccharides and polysaccharides. Specifically, this class includes sugar-based binders such as sucrose, high-fructose corn syrup, molasses and polydextrose. Additionally, starches, gums, and celluloses may be utilized as food-grade binders. Molasses and high-fructose corn syrup are particularly preferred food-grade binders because the heat treatment step of the process of this invention allows these binders to be pyrolyzed to form relatively insoluble water resistant agglomerates.

The invention also provides a low density, substantially water-insoluble, calcium carbonate-containing, particulate material prepared by a process as described above and having a particle size of from about 5 to about 300 microns, a particle density of not more than 1.0 g/ml. and a resistance against degradation in water.

A preferred particulate material according to the invention is one having a particle size of from about 5 to about 300 microns and a particle density of 0.7 to 0.9 g/ml.

Preferably the particulate material comprises about 10 to 20 weight percent carbohydrate-based binder and the balance calcium carbonate.

As used herein, the expression "resistance to degradation in water" means that the particulate material oragglomerate of the present invention retains its integrity after prolonged contact with water and an evaluation of this characteristic is presented in the experimental results appearing hereinafter.

The low density particulate material of the present invention is useful as a filler or bulking agent in polymers, paper, construction materials or other applications where a lightweight bulking agent is desired. It is particularly useful in certain food products where a lightweight aggregate of food-grade quality is desired.

The low density agglomerate of the present invention is a porous medium which is also useful as a carrier for fragrances, flavors, insecticides, fertilizers and catalysts, for applications in the cosmetics, agricultural and chemical fields. The porosity of the agglomerate makes it useful as an absorbant.

As indicated hereinabove, calcium carbonate has been widely used as a filler or bulking agent in various industrial products, in pharmaceuticals and in food. However, there are some applications wherein a material ofrelatively low density is required and conventional calcium carbonate, having a density of 2.7 to 2.83 g/ml.,

is not suitable.

Since calcium carbonate has many desirable chemical and physical properties which render it suitable as a bulking agent and also it is readily available and easy to purify, it was considered worthwhile to ascertain whether it could be subjected to a density-reducing treatment whereby the resulting product, while retaining the normal advantages associated with calcium carbonate, would be suitable for those applications wherein a low density material is essential or desirable.

The desired lightweight agglomerates may be obtained by adding a suitable carbohydrate binder to precipitated calcium carbonate, followed by heat treatment. Experiments have established that carbohydrate binders such as molasses and corn syrup produce low density agglomerates when added to precipitated calcium carbonate and subjected to this process. These agglomerates are resistant to degradation in water when heated to 300 to 450°C for 2 hours. For many applications carbohydrates, including gums, starches, sugars, cellulose, hemicelluloses, etc. can be used to obtain porous, low density agglomerates that are resistant to degradation in water.

In a preferred embodiment of the invention, about 10 to 20 percent by weight (dry solids) of an appropriate carbohydrate binder is admixed with synthetic calcium carbonate. Albacar brand calcium carbonate is preferably used because the unique morphology of the particles is especially beneficial in contributing to low density. The carbohydrate binder may be selected from the group consisting of sugar-based binders such as sucrose, high fructose corn syrup, molasses and polydextrose ; saccharides and polysaccharides ; starches ; gums ; and celluloses and hemicelluloses. Molasses and corn syrup are preferred.

The carbohydrate binder is added to the calcium carbonate and the resulting slurry is mixed vigorously by hand or with a mechanical mixer until a uniform consistency is achieved ; indicating homogeneous distribution of the carbohydrate-based binder throughout the calcium carbonate. Low density agglomerates are made by spray drying the slurry at appropriate conditions to obtain a particle size from about 300 microns to near ultimate particle size, as desired. This material is then screened and fired in an oxygen-free atmosphere at 200 to 800°C, preferably 300 to 600°C, to make a water resistant product. After cooling, the particulate material is crushed and screened.

In an alternative embodiment, the slurry is first partially dried to a mixture having a damp consistency. The damp mixture is pressed into disks, for example, by using a hydraulic press. A suitable size for said disks is about 8cm. in diameter. The pressure is controlled to produce disks of a desired strength and density. The disks are then crushed into granules and the resulting particulate material is fired at a temperature of about 200 to 800°C., preferably about 300 to 600°C., in either a gas or an electric kiln, and soaked for about one hour. After cooling the particulate material is crushed to the desired particle size for a given application. For example, an aggregate of food-grade quality may be prepared with a particle size in the range of from about 5 to about 300 microns.

Drying of the agglomerate can be accomplished according to the present invention by a number of means known in the art, including, but not limited to, static bed drying, fluid bed drying and spray drying.

In a further alternative embodiment of the process of this invention, the disk is dried, crushed and screened to a particle size of from at least about +400 mesh (U.S. Standard Sieve), corresponding to from about 37 microns and larger, prior to heating of the dried material, instead of crushing and screening the material after heating.

When the required particle size is larger than 300 microns, a pelletizing device is used to obtain the desired size fraction of low density agglomerate.

Other sizing techniques, including air classification and elutriation, can be utilized as alternatives to crushing and screening. Air classification is utilized where it is desired to maintain the agglomerate in a dry state and elutriation is utilized where the agglomerate can be separated in a liquid medium.

The following Examples illustrate preferred embodiments of the process of the invention and the resulting lightweight agglomerates. These Examples are non-limiting. Other examples and applications within the scope of the appended claims will be evident to those skilled in the art.

Example 1

Large Batch with 15% Molasses by Weight (molasses solids to PCC solids)

Five hundred seventeen (517) pounds of food-grade calcium carbonate (Albacar 5971, Pfizer, N.Y.) having an average particle size of about 1.9 microns was added to 653 lbs of water containing 183 lbs of molasses (50% sugar solids) in a stainless steel vessel. The slurry with 45% solids was mixed well with a high shear motor driven mixer until a smooth, homogeneous consistency was obtained. The slurry was then fed to a spray dryer at an appropriate feed rate and temperature required to leave less than 2% moisture. The spherical agglomerates obtained were screened to -120 +230 mesh (U.S. Standard Sieve) and individual samples were heated at 300°C, 400°C, and 450°C for 2 hours. The resulting products comprised discreet particles of calcium carbonate having a bulk density of 0.53 g/cc. Based on the standard void volume of 40% for closely sized spheres the sphere density was estimated to be about 0.88 g/cc. See Table I. From this the pore volume of the particles is calculated to be approximately 61%. The product showed good particle integrity, low dusting and was resistant to degradation in water as the heat treatment temperature was increased to 450°C. See Table II. The product was light grey-brown and it was free flowing in nature.

Example 2

Large Batch with 15% Corn Syrup by Weight (syrup solids to PCC solids

Five hundred seventy (570) pounds of food-grade calcium carbonate (Albacar 5971) having an average prticle size of 1.9 microns was added to 789 lbs of water contaning 131 lbs of high fructose corn syrup (77% sugar solids) in a stainless steel vessel. This slurry with 45% solids was mixed well with a high shear motor driven mixer until a smooth homogeneous consistency was obtained. The slurry was then fed to a spray dryer at an appropriate feed rate and temperature required to leave less than 2% moisture. The sperical agglomerates obtained were screened to -120 +230 mesh and then heated at 300°C, 350°C, 400°C, and 450°C for 2 hours. The resulting products comprised discreet particles of calcium carbonate having a bulk density of 0.5 g/cc. Based on the standard void volume of 40% for closely sized spheres the sphere density was estimated to be about 0.85 g/cc. See Table II. From this value the pore volume is calculated to be approximately 65%. This product showed low dusting, good particle integrity and was relatively resistant to degradation in water as the heat treatment temperature was increased to 450°C. See Table I. The product was light grey-brown and free flowing in nature.

Table I

Density Values of Various Low Density Agglomerate Samples

| Thermal Treatment | Example 1 (Molasses) | | | Example 2 (Corn Syrup) | | |
|---|---|---|---|---|---|---|
| | Real | Density (g/ml) Bulk | Density (g/ml) Agglomerate[1] | Real | Density (g/ml) Bulk | Density (g/ml) Agglomerate[1] |
| 300°C at 2 hrs | 2.16 | 0.53 | 0.88 | 2.4 | 0.53 | 0.88 |
| 350°C at 2 hrs | - | 0.53 | 0.89 | - | 0.49 | 0.81 |
| 400°C at 2 hrs | - | 0.52 | 0.87 | - | 0.48 | 0.80 |
| 450°C at 2 hrs | 2.31 | 0.53 | 0.88 | 2.34 | 0.49 | 0.81 |

[1] Agglomerate density is estimated from the assumption that there is 40% void volume for closely sized packed spheres.

Table II

Percentage of -120 +230 mesh (U.S. Standard Sieve) (66 to 125 microns) Product Retained after Heat Treatment plus Dry and Wet Screening

| | Example 1 (Molasses) | | Example 2 (Corn Syrup) | |
|---|---|---|---|---|
| Thermal Treatment | Dry | Wet | Dry | Wet |
| 2 hours at 300°C | 94 | 83 | 92 | 72 |
| 2 hours at 350°C | 96 | 84 | 94 | 75 |
| 2 hours at 400°C | 97 | 87 | 95 | 76 |
| 2 hours at 450°C | 97 | 91 | 97 | 87 |

## Claims

1. A low density, substantially water-insoluble particulate material characterized by precipitated calcium carbonate and up to about 40% by weight, based on the weight of calcium carbonate,of a carbohydrate binder selected from the group consisting of molasses, corn syrup, sugars, starches, gums cellulose, and hemicelluloses, having a particle size of from about 5 to about 300 microns, and a particle density of no more than 1.0 g/ml.

2. The particulate material according to claim 1 further characterized in that the carbohydrate binder is from about 2 to about 25 weight percent of the total weight.

3. The particulate material according to claim 1 further characterized in that the density is from about 0.7 to about 0.9 g/ml.

4. The particulate material according to claim 2 further characterized in that the carbohydrate binder is molasses.

5. A process for the preparation of a low density, substantially water-insoluble, particulate material characterized by forming an aqueous slurry containing precipitated calcium carbonate having an initial average particle size of about 0.5 to 4.0 microns and 0 to 40% by weight, based on the weight of the calcium carbonate, of a dissolved carbohydrate binder, drying the slurry to provide a granular material, sizing the granular material to a particle size of from about 5 to about 300 microns, heating the dried material to a temperature within the range of 200° to 800°C in the absence of oxygen and maintaining the temperature for a period of one to two hours, cooling the resulting agglomerate and crushing the material to a particle size of from about 5 to about 300 microns, to provide the desired particulate material having a particle density of no more than 1.0 g/ml. and a resistance against degradation in water.

6. The process according to claim 5 further characterized by the carbohydrate binder being one selected from the group consisting of molasses, corn syrup, sugars, starches, gums cellulose, and hemi-celluloses.

7. The process according to claim 6, further characterized by the carbohydrate binder being molasses.

8. The process according to claim 5 further characterized by the heating step being carried out at a temperature within the range of 300° to 600°C.

9. The process according to claim 5 further characterized by drying of the slurry being performed using means selected from the group consisting of static bed drying, fluid bed drying and spray drying.

10. The process according to claim 9 further characterized by the condition that when the means selected for drying the slurry is spray drying, the crushing step is deleted.

11. A low density, substantially water-insoluble, calcium carbonate-containing, particulate material characterized by being prepared according to the process of claim 5 and having a particle size of from about 5 to about 300 microns, a particle density of not more than 1.0 g/ml. and a resistance against degradation in water.

12. A particulate material according to claim 9 characterized by containing from about 2 to about 25 weight percent carbohydrate binder and the balance calcium carbonate, and having a particle size of from about 5 to about 300 microns and a particle density of 0.7 to 0.9 g/ml.

European Patent Office

# EUROPEAN SEARCH REPORT

Application Number

EP 91300605.2

| Category | Citation of document with indication, where appropriate, of relevant passages | Relevant to claim | CLASSIFICATION OF THE APPLICATION (Int. Cl.5) |
|---|---|---|---|
| X | US - A - 4 744 987 (MEHRA et al.) * Column 2, line 33 - column 4, line 42; column 5, lines 11-34 * -- | 1,2,5, 6,8-12 | C 09 C 1/02 //A 61 K 47/02 A 61 K 9/16 C 08 K 9/08 C 08 K 3/26 D 21 H 19/44 |
| D,P, A | EP - A1 - 0 386 868 (PFIZER INC.) * Claims 1,4-14 * -- | 1,3,5, 8-11 | |
| A | EP - A2 - 0 344 984 (PFIZER INC.) * Claims 1,4,5,8 * -- | 1,2,5, 6 | |
| A | GB - A - 1 425 114 (THE ASSOCIATED PORTLAND CEMENT MANUFACTURERS LIMITED) * Page 1, lines 49-71 * ---- | 5,6 | |
| | | | TECHNICAL FIELDS SEARCHED (Int. Cl.5) |
| | | | C 09 C A 61 K C 08 K D 21 H |

The present search report has been drawn up for all claims

| Place of search | Date of completion of the search | Examiner |
|---|---|---|
| VIENNA | 06-05-1991 | HAUSWIRTH |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)